Europäisches Patentamt

European Patent Office

Office européen des brevets

(19)

(11) Veröffentlichungsnummer: **0 378 086 B1**

(12) **EUROPÄISCHE PATENTSCHRIFT**

(45) Veröffentlichungstag der Patentschrift: **26.10.94**

(51) Int. Cl.5: **A61K 31/55**

(21) Anmeldenummer: **90100071.1**

(22) Anmeldetag: **03.01.90**

Teilanmeldung 92119073.2 eingereicht am 06/11/92.

(54) **Verwendung des Wirkstoffs Azelastin zur Bekämpfung von Psoriasis-Erkrankungen.**

(30) Priorität: **11.01.89 DE 3900607**

(43) Veröffentlichungstag der Anmeldung:
**18.07.90 Patentblatt 90/29**

(45) Bekanntmachung des Hinweises auf die
Patenterteilung:
**26.10.94 Patentblatt 94/43**

(84) Benannte Vertragsstaaten:
**AT BE CH DE DK ES FR GB GR IT LI LU NL SE**

(56) Entgegenhaltungen:
**DE-A- 2 164 058**

**JPN. J. CLIN. OPHTHALMOL., Band 42, Nr. 7, 1988, Seiten 781-784; M. KONDO etal.: "Ouantitation of inflammation in the anterior chamber after cataractsurgery"**

**BR. J. PHARMACOL., Band 87, Nr. 2, 1986, Seiten 443-448, The Macmillan Press Ltd; N. CHAND et al.: "Modulation of in vitro anaphylaxis of guinea-pigisolated tracheal segments by azelastine, inhibitors of arachidonic acidmetabolism and selected antiallergic drugs"**

(73) Patentinhaber: **ASTA Medica Aktiengesellschaft**
**An der Pikardie 10**
**D-01277 Dresden (DE)**

(72) Erfinder: **Engel, Jürgen, Dr.**
**Erlenweg 3**
**D-8755 Alzenau (DE)**
Erfinder: **Mollière, Michael, Dr.**
**de Neufville 8**
**D-6000 Frankturt 70 (DE)**
Erfinder: **Szelenyi, Istvan, Dr.**
**Händelstrasse 32**
**D-8501 Schwaig (DE)**

Rank Xerox (UK) Business Services
(3.10/3.09/3.3.3)

ALLERGY, Band 41, 1986, Seiten 473-478; N. CHAND et al.: "Inhibition ofleukotriene (SRS-A)-mediated acute lung anaphylaxis by azelastine in guineapigs"

BIOCHEMICAL PHARMACOLOGY, Band 33, Nr. 20, 1984, Seiten 3165-3169, Pergamon-Press Ltd, GB; K. TANIGUCHI et al.: "Inhibitory effects of various drugs onphorbol myristate acetate and n-formyl methionyl-leucyl phenylalanine induced02 production in polymorphonuclear leukocytes"

PROSTAGLANDINS, Band 32, Nr. 4, Oktober 1986, Seiten 481-494; J.G. MASSICOT etal.: "Potential therapeutic uses of inhibitors of leukotriene generation andfunction. Session I. Pharmacologic regulation of the leukotrine pathway"

J. Jpn. Soc. Colo-Procol. Band 42(5), Seite 916, 1989

## Beschreibung

Der Arzneimittelwirkstoff Azelastin (chemische Bezeichnung 4-(p-chlor-benzyl)-2-(hexahydro-1-methyl-1H-azepin-4-yl)-1(2H)-phthalazinon) ist ein Asthmaprophylaktikum und Antiallergikum (siehe deutsche Patentschrift 21 64 058).

Es wurde nun gefunden, daß Azelastin auch gegen Psoriasis und hiermit verwandte Erkrankungen wirksam ist.

Die Erfindung betrifft die Verwendung von Azelastin zur Herstellung von Mitteln zur Bekämpfung von Psoriasis-Erkrankungen wie Psoriasis und psoriasisverwandten Erkrankungen.

Unter Psoriasis-Erkrankungen werden im Sinne der Erfindung Hauterkrankungen verstanden, die mit Hyperkeratosen einhergehen wie insbesondere Psoriasis.

Wirkung von Azelastin bei Psoriasis-Erkrankungen und hiermit verwandten Erkrankungen (beispielsweise Psoriasis) und Prüfung dieser Wirkung.

Mäuse (Durchschnittsgewicht 25 g) erhielten 7 Tage lang täglich 1 mg/kg Azelastin oral. Am 8. Tag wurde die obere Hautschicht mit Sandpapier entfernt. Durch diese mechanische Irritation und Entfernung der obersten Hautschicht entsteht eine akute Reaktion, die morphologische Ähnlichkeiten zur psoriatischen Reaktion aufweist. Außerdem erhöht sich der Leukotrien-Gehalt in der Dermis und Epidermis. Dies geht mit einer Erniedrigung der Prostaglandin-Konzentration einher. Auch diese Veränderungen sind für Psoriasis typisch. Durch die Vorbehandlung mit Azelastin fanden diese Veränderungen nicht statt. Azelastin normalisiert beziehungsweise erniedrigt sogar die dermale beziehungsweise epidermale Leukotrien-Konzentration und erhöht die Konzentration der Prostaglandine.

Zur Behandlung von Psoriasis werden zur Zeit hauptsächlich Corticosteroide eingesetzt. Demgegenüber hat das Azelastin nur geringe beziehungsweise zu vernachlässigende Nebenwirkungen und hemmt die Leukotrien-Synthese in der Haut auch nach systemischer Gabe; darüberhinaus tritt durch Azelastin auch eine Hemmung der durch PAF (Platelet activating factor) induzierten Ödembildung ein.

Die pharmazeutischen Zubereitungen für die Anwendung bei Psoriasis-Erkrankungen und hiermit verwandten Erkrankungen am Menschen enthalten im allgemeinen zwischen 2 bis 16 vorzugsweise 4 bis 8 mg Azelastin.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsform kommen zum Beispiel in Frage: Ölige oder alkoholische beziehungsweise wässrige Lösungen sowie Suspensionen und Emulsionen. Bevorzugte Anwendungsformen sind Tabletten, die zwischen 4 und 8 mg oder Lösungen, die zwischen 0,1 bis 10 Gewichtsprozent an Azelastin enthalten.

Die Einzeldosis von Azelastin für die Anwendung bei Psoriasis-Erkrankungen und hiermit verwandten Erkrankungen kann beispielsweise liegen:

a) bei oralen Arzneiformen zwischen 2 und 16 mg vorzugsweise 8 mg;
b) bei Arzneiformen zur lokalen Applikation auf die Haut und Schleimhäute (zum Beispiel in Form von Lösungen, Lotionen, Emulsionen, Salben und so weiter) zwischen 0,1 und 10 Gewichts%, vorzugsweise 0,7 - 5 Gewichts%, insbesondere 1 - 5 Gewichts%.

Beispielsweise können 3mal täglich 1 bis 2 Tabletten mit einem Gehalt von 4 bis 8 mg Azelastin empfohlen werden. Bei oraler Verabreichung ist die minimale tägliche Dosis beispielsweise 4 mg; die maximale tägliche Dosis bei oraler Verabreichung soll nicht über 24 mg liegen.

-(Die Dosen sind jeweils bezogen auf die freie Base)-

Die akute Toxizität von Azelastin an der Maus (ausgedrückt durch die LD 50 mg/kg; Methode nach Miller und Tainter: Proc. Soc. Exper. Biol a. Med. <u>57</u> (1944) 261) liegt beispielsweise bei oraler Applikation zwischen 120 und 170 mg/kg.

-(Die Dosen sind jeweils bezogen auf die freie Base)-

Die pharmazeutischen Zusammensetzungen beziehungsweise Arzneimittel enthalten als Wirkstoff Azelastin oder seine physiologisch verträglichen Salze. Der Wirkstoff liegt gegebenenfalls in Mischung mit anderen pharmakologisch beziehungsweise pharmazeutisch wirksamen Stoffen vor. Die Herstellung der Arzneimittel erfolgt in bekannter weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 und ff.; H.v.Czetsch-Lindenwald, Hilfsstoffe für Pharmazie und angrenzende Gebiete; Pharm. Ind., Heft 2 (1961), Seite 72 und ff.; Dr. H.P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Polyvinylacetat, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalko holen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl , Rizinusöl , Olivenöl, Sesamöl, Baumwollsaatöl , Maisöl , Weizenkeimöl Sonnenblumensamenöl , Kabeljau-Leberöl, jeweils auch hydriert; Mono-, Di- und Triglyceride von gesättigten Fettsäuren $C_{12}H_{24}O_2$ bis $C_{18}H_{36}O_2$ und deren Gemische, pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden. Als solche kommen beispielsweise in Frage: Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (zum Beispiel Eudragit® RS), Copolymerisate aus Acryl- und Methacrylsäureestern und Trimethylammoniummethacrylat (zum Beispiel Eudragit® RL); Polyvinylacetat; Fette, Öle, Wachse, Fettalkohole; Hydroxypropylmethylcellulosephthalat oder -acetatsuccinat; Cellulose-, Stärke- sowie Polyvinylacetatphthalat; Carboxymethylcellulose; Methylcellulose-phthalat, -succinat, -phthalatsuccinat sowie -phthalatsäurehalbester; Zein; Ethylcellulose sowie -succinat; Schellack, Gluten; Ethylcarboxyethylcellulose; Ethacrylat-Maleinsäureanhydrid-Copolymer; Maleinsäurenanhydrid-Vinylmethylether-Copolymer; Styrol-Maleinsäure-Copolymerisate; 2-Ethyl-hexylacrylatmaleinsäureanhydrid;Crotonsäure-VinylacetatCopolymer; Glutaminsäure/Glutaminsäureester-Copolymer; Carboxymethylethyl-celluloseglycerinmonooctanoat; Celluloseacetatsuccinat; Polyarginin.

Als Plastifizierungsmittel für Hüllstoffe können in Frage kommen:
Citronen- und Weinsäureester (Acetyltriethyl-, Acetyltributyl-, Tributyl-, Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-phthalat), D-(2-Methoxy- oder ethoxyethyl)-phthalat, Ethylphthalyl-, Butylphthalylethyl- und Butylglycolat; Alkohole (Propylenglycol, Polyethylenglycol verschiedener Kettenlängen), Adipate (Diethyl-adipat, Di(2-Methoxy- oder ethoxyethyladipat); Benzophenon; Diethyl- und Dibutylsebacat, -succinat, -tartrat; Diethylenglycoldipropionat; Ethylenglykoldiacetat, -dibutyrat, -dipropionat; Tributylphosphat, Tributyrin;
Polyethylenglykolsorbitanmonooleat (Polysorbate wie Polysorbat 80); Sorbitanmonooleat.

Zur Herstellung von Lösungen oder Suspensionen kommen beispielsweise Wasser oder physiologisch verträgliche organische Lösungsmittel in Frage, wie zum Beispiel Ethanol, Propanol, Isopropanol, 1,2-Propylenglykol, Polyglykole und deren Derivate, Dimethylsulfoxid, Fettalkohole, Triglyceride, Partialester des Glycerins, Paraffine und ähnliche.

Für injizierbare Lösungen oder Suspensionen kommen zum Beispiel nicht-toxische parenteral vertragliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage:
Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisterte Oleotriglyceride, Polyethylenoxyd-Kondensationspro-

EP 0 378 086 B1

dukte von Fettalkoholen, Alkylphenolen oder Fettsäuren oder auch 1-Methyl-3-(2-hydroxyethyl)imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro Mol Glycerid).

Beispiele für Oleotriglyceride sind Olivenöl, Erdnußöl, Rizinusöl, Sesamöl, Baumwollsaatöl, Maisöl. Siehe auch Dr. H. P. Fiedler "Lexikon der Hilfsstoffe für Pharmazie, Kosmetik und angrenzende Gebiete" 1971, S. 191-195.

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Zur Herstellung von dermal anzuwendenden Zubereitungen kommen in Frage die vorgenannten Substanzen und stretchfähige oder flüssige Kohlenwasserstoffe wie Vaselin oder Paraffin oder Gele aus Paraffinkohlenwasserstoffen und Polyethylen, Fette und Öle pflanzlichen oder tierischen Ursprungs, die zum Teil auch hydriert sein können oder synthetische Fette wie Glyceride der Fettsäuren $C_8$-$C_{18}$, sodann Bienenwachs, Cetylpalmitat, Wollwachs, Wollwachsalkohole; Fettalkohole wie Cetylalkohol, Stearylalkohol, Polyethylenglykole vom Molekulargewicht 200 bis 20 000; flüssige Wachse wie Isopropylmyristat, Isopropylstearat, Äthyloleat; Emulgatoren wie Natrium-, Kalium-, Ammoniumsalze der Stearinsäure oder Palmitinsäure sowie Triethanolaminstearat, Alkalisalze der Ölsäure, Ricinolsäure, Salze von sulfurierten Fettalkoholen wie Natriumlaurylsulfat, Natriumcetylsulfat, Natriumstearylsulfat, Salze der Gallensäure, Sterole wie Cholesterol, Partialfettsäureester mehrwertiger Alkohole wie Ethylenglykolmonostearat, Glycerolmonostearat, Pentaerythritmonostearat, Partialfettsäureester des Sorbitans, Partialfettsäureester des Polyoxyethylensorbitans Sorbitolether des Polyoxyethylens, Fettsäureester des Polyoxyethylens, Fettalkoholether des Polyoxyethylens, Fettsäureester der Saccharose, Fettsäureester des Polyglycerols, Lecithin.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisteren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan. Bei den parenteralen Zubereitungsformen handelt es sich insbesondere um sterile beziehungsweise sterilisierte Erzeugnisse.

Zur Behandlung der Psoriasis wird Azelastin lokal, zum Beispiel in Form von Lösungen, Tinkturen, Suspensionen, Emulsionen, Salben, Gelen, Cremes, Pasten, Lotionen oder Shampoos eingesetzt. Bevorzugt werden wasserfreie Zubereitungen verwendet, wodurch die gleichzeitige Anwendung von Saltcylsäure und/oder Harnstoff ermöglicht wird. Derartige Zubereitungen, die durch den Zusatz von Tensiden auch abwaschbar gemacht werden können, sind beispielsweise in der Deutschen Offenlegungsschrift 36 03 859 beschrieben. Der Harnstoff kann entweder als Tensid-Harnstoff-Einschlußverbindung oder auch in freier Form vorliegen. Zubereitungen, die weder Harnstoff noch Salicylsäure enthalten, können selbstverständlich auch Verwendung finden.

Die Einsatzkonzentrationen an Azelastin betragen hierbei beispielsweise 0,1 bis 10 % (Gewicht/Gewicht), vorzugsweise 0,5 bis 8 %, insbesondere 1 bis 5 %. Die Einsatzkonzentrationen an Salicylsäure betragen beispielsweise 0,1 bis 10 %, vorzugsweise 0,2 bis 8 %, insbesondere 0,5 bis 5 %.

5

Die Einsatzkonzentrationen an Harnstoff betragen beispielsweise 1 bis 20 %, vorzugsweise 3 bis 18 %, insbesondere 5 bis 15 %.

1. Azelastin-Filmtabletten für orale Applikation

Herstellung der Tabletten

Aus 440 g Azelastin-HCl, 360 g mikrokristalliner Cellulose und 200 g Talkum wird eine Vormischung hergestellt. Diese Vormischung sowie 6000 g Lactose-Monohydrat, 2870 g mikrokristalline Cellulose und 100 g hochdisperses Siliciumdioxid werden durch ein Sieb gegeben und in einem geeigneten Mischer homogenisiert. Zu der so erhaltenen Mischung werden 30 g Magnesiumstearat zugesiebt und das Ganze noch einmal homogenisiert. Die so erhaltene Masse wird zu Tabletten von 100 mg Gewicht, 6 mm Durchmesser und einem Wölbungsradius von 5,5 mm verpreßt.

Herstellung der Filmtabletten

In einem hierfür üblichen Gerät werden die Tabletten mit 1,2 kg einer Filmsuspension kontinuierlich besprüht:

Zur Herstellung der Filmsuspension werden 60 g Polyethylenglykol 6000, 12 g Polysorbat 80 und 9,6 g Carboxymethylcellulose-Natrium in 787,2 g Wasser gelöst. In dieser Lösung werden 120 g Talkum, 120 g Titandioxid und 1,2 Simethicone*dispergiert und anschließend unter leichtem Rühren 90 g eines Copolymerisats auf Basis von Dimethylaminoethylmethacrylat und neutralen Methacrylsäureestern in Form einer 30%igen wässrigen Dispersion (Eudragit® E 30 D), zugefügt.

1 Filmtablette enthält 4 mg Azelastin (entsprechend 4,4 mg Azelastinhydrochlorid).

2. Azelastin-8-mg-Filmtabletten

Herstellung von Film-Tabletten

88 g Azelastinhydrochlorid, 701 g Lactose-Monohydrat und 701 g mikrokristalline Cellulose werden gesiebt, gemischt und mit 1440 g einer ph-unabhängigen Acrylharzlacksubstanz, die schwer durchlässige Überzüge bildet (Eudragit® RS 12,5) angefeuchtet. Die feuchte Masse wird durch ein Sieb granuliert und getrocknet.

Das trockene Granulat, 100 g Talkum und 30 g Magnesiumstearat werden erneut gesiebt und in einem geeigneten Mischer homogenisiert. Diese Masse wird zu Tabletten von 180 mg Gewicht, 8 mm Durchmesser und einem Wölbungsradius von 6 mm verpreßt.

Zur Herstellung der Filmtabletten werden die Tabletten in einem hierfür üblichen Gerät mit einer Filmsuspension besprüht, die wie folgt hergestellt wird: In 664 g Isopropanol werden unter Rühren 18 g Magnesiumstearat dispergiert; in diese Suspension werden dann 18 g 1,2 Propylenglykol und 800 g eines anionischen Polymerisats aus Methacrylsäure und Methacrylsäureestern, das einen Weichmacher enthält, magensaftresistent ist und erst ab pH 7 löslich ist (Eudragit S 12,5), eingearbeitet.

1 Filmtablette enthält 8 mg Azelastin (entsprechend 8,8 mg Azelastinhydrochlorid).

3. Azelastin 8-mg-Kapseln

100 g Azelastinhydrochlorid, 200 g Weinsäure, 500 g Milchzucker und 700 g mikrokristalline Cellulose werden gemischt und mit etwa 700 g gereinigtem Wasser angeteigt. Die feuchte Masse wird durch ein Lochblech mit einem Lochdurchmesser von 1 mm gepreßt und die entstehenden Stränge durch Behandlung auf einer Spheronizer-Scheibe in üblicher Weise zerteilt und ausgerundet. Die erhaltenen Pellets werden getrocknet und gesiebt.

1000 g Pellets der Siebfraktion 800 bis 1250 μm werden mit einer Suspension besprüht, die folgendermaßen hergestellt wird:

In 190 g gereinigtem Wasser werden 0,6 Polysorbat 80 gelöst und in der Lösung 40 g Triethylcitrat emulgiert. Zu der erhaltenen Emulsion werden 800 g einer 30%igen wässrigen Dispersion eines Copolymerisates aus Acryl- und Methacrylsäureestern mit einem geringen Gehalt an Trimethylammoniummethacrylatchlorid (= Eudragit® RS 30 D) zugegeben und etwa 10 Minuten lang gerührt.

In 860 g gereinigtem Wasser werdedn 109,2 g Talkum und 0,2 g Silikon-Antischaumöl (Simethicone) suspendiert. Diese Suspension wird in die oben erhaltene Dispersion eingerührt.

Der Auftrag der damit erhaltenen Lackier-Suspension auf die Pellets geschieht in üblicher Weise, zum Beispiel unter Verwendung eines Wirbelschichtsprühgranulators bei einer Zulufttemperatur von 40-50°C und einer Ablufttemperatur von maximal 40°C. Die Trocknung der Pellets erfolgt unter den gleichen Bedingungen.

Es wird so viel von oben genannter Lackier-Suspension aufgesprüht, bis das Gesamtgewicht der getrockneten Pellets 1127 g beträgt.

*) Simethicone ist eine Mischung aus Polydimethylsiloxan (Vorwiegend 90-99 Gewichts%) und Siliciumdioxid (beispielsweise 4-7 Gewichts%); US-Pharmakopoe, 21. Ausgabe

Die lackierten Pellets werden in einer Menge von 148,7 mg in Hartgelatinekapseln der Größe 1 gefüllt. Eine Hartgelatinekapsel enthält 8,8 mg Azelastinhydrochlorid entsprechend 8 mg Azelastin in Retardzubereitung. Die Freisetzung von Azelastinhydrochlorid aus dieser Darreichungsform wird geprüft nach dem Verfahren der US-Pharmakopoe, 21. Ausgabe (USP XXI) mit dem Gerät für den Dissolution-Test, Apparatur 2. Bei einer Umdrehungsgeschwindigkeit des Paddles von 100 Umdrehungen pro Minute wird die Freisetzung des Azelastinhydrochlorid in 500 ml Prüflösung bei 37°C bestimmt. Die Prüflösung besteht für die ersten 2 Stunden aus 0,1 molarer Salzsäure, danach werden die Pellets in Phosphatpufferlösung pH 6,8 der Europäischen Pharmakopoe überführt. Aus den Prüflösungen wird jeweils die Freisetzung des Azelastinhydrochlorid gemessen.

Die Freisetzung beträgt

| | | | | |
|---|---|---|---|---|
| nach | 1 | Stunde | 5,5 % | in 0,1 m HCl |
| nach | 2 | Stunden | 8,4 % | |
| nach | 3 | Stunden | 11,2 % | |
| nach | 4 | Stunden | 14,3 % | |
| nach | 5 | Stunden | 21,7 % | |
| nach | 6 | Stunden | 31,7 % | |
| nach | 7 | Stunden | 39,2 % | in Phosphat- |
| nach | 8 | Stunden | 48,5 % | puffer pH 6,8 |
| nach | 9 | Stunden | 56,1 % | |
| nach | 10 | Stunden | 64,0 % | |
| nach | 11 | Stunden | 74,5 % | |
| nach | 12 | Stunden | 81,2 % | |

**Patentansprüche**

1. Verwendung von Azelastin oder dessen therapeutisch verwertbaren Salzen für die Herstellung eines Arzneimittels zur Bekämpfung von Psoriasis-Erkrankungen

**Claims**

1. The use of azelastine or therapeutically acceptable salts thereof for the production of a medicament for treating psoriasis diseases.

**Revendications**

1. Utilisation de l'azélastine ou de ses sels utilisables en thérapeutique pour la préparation d'un médicament pour le traitement du psoriasis.